# EUROPEAN PATENT APPLICATION

(11) **EP 3 214 047 A1**
(43) Date of publication of application: **06.09.2017**
(21) Application number: 17275028.3
(22) Date of filing: 01.03.2017
(51) Int. Cl.: C02F 1/48

(54) **SYSTEM AND METHOD FOR IMPARTING ELECTROMAGNETIC ENERGY INTO WATER AND USE THEREOF**

(30) Priority: 03.03.2016 US 201662302951 P
(71) Applicant: LIFENG TECHNOLOGY LIMITED, Kwun Tong, Kowloon (HK)
(72) Inventor: Foo, Onn Fah, Kowloon (HK)
(74) Representative: Potter Clarkson LLP

(57) **Abstract**

The present invention provides a system for imparting electromagnetic energy into drinking water or aqueous potables and foods, comprising: a generator unit for generating a driving signal having a frequency to maintain a nature or an activity of microorganisms in living forms; a power supply unit for providing a voltage current to the generator unit to drive the generation of the driving signal; one or more inductor coils coupled to the generator unit to receive the driving signal, such that an electromagnetic field operating with the varying frequency is generated; and an electromagnetic wave-permeable container for containing the drinking water or the aqueous potables and foods wherein the drinking water or the aqueous potables and foods is subject to the generated electromagnetic field to allow permeation of the varying frequency electromagnetic wave through the container into the drinking water or the aqueous potables and foods to acquire electromagnetic energy. The invention also provides a method for imparting electromagnetic energy into drinking water or aqueous potables and foods.

## Description

### Field of the Invention

This invention relates generally to the field of energy acquisition in a fluid, and more particularly, to system and method for imparting electromagnetic energy into drinking water or aqueous potables and foods, by application of varying electromagnetic wave operating in a desired frequency. The invention also relates to a method of using the treated drinking water or aqueous potables and foods to allow a mammalian body to acquire the energy required for various physiological processes such as metabolism process.

### Background of the Invention

Electromagnetic waves find a wide range of applications in different areas, such as environment protection, industrial and biological applications. US patent application no. US 2002/0009797 filed by the US National Aeronautics and Space Administration (NASA) in 2001 discloses use of electromagnetic wave to promote tissue growth and bone regeneration and teaches to grow the cells within a culture medium facilitated by an electromagnetic field. Another US patent application no. US 2014/0342428A1 filed by NASA discloses methods to modify the genetic regulation of mammalian tissues, bones, cells by electromagnetic stimulation fields.

Over the past few decades, people concerns about their health increase, and medical, pharmaceutical and nutrition industry has shown interest in the research of the treatment and/or prevention of various diseases as well as in maintaining good health and physical function.

Metabolism is a continuous and functioning process, one of the important physiological processes of the human body. It embodies all chemical reactions occurring in the body to convert food, water and oxygen intakes into energy and products the various synthesis processes required to sustain life. Metabolism keeps alive all living forms of microorganisms on earth. In fact, all metabolic processes in animals and plants are chemical reactions and all require energy for the reactions to proceed and take place in the cells. Metabolism is a chemical reaction, and two factors determine whether the metabolic process will proceed: heat energy (enthalpy) and molecular (vibrational) internal energy (entropy). The heat energy which the body relies on to drive the metabolic process is derived from the intake of food, through this process, only heat energy is produced, while no vibrational energy is produced.

In the metabolism process, plant draws electromagnetic wave energy from sunlight for their metabolism process to proceed. In the process, sunlight electromagnetic wave energy excites the chlorophyll in chloroplast to convert water into energy in the form of ATP and NADPH which further convert into sugar in the Calvin cycle by taking in CO₂. In this process, the energy from metabolic reaction actually comes from the sunlight electromagnetic wave energy.

For animals, the metabolic energy comes from the food intake instead of sunlight. In animal metabolism, it consists of catabolism and anabolism. In catabolism, food intakes such as carbohydrates, fats, or proteins are broken down into small simple bio-molecules such as Glucose, Fatty acid, Amino acid as well as the conversion into ATP in the mitochondria inside the cells. The ATP provides the energy needed by the animals for its body activities including the energy needed by the body anabolic process for building up new cells from the small bio molecules broken down in the catabolic process.

Both plants and animal metabolic processes are very similar and they all go through the process to produce ATP for the energy they required. However, in all their processes, the energy can also be stored in the form of glucose, fat or protein in their body and they may be used when there is no sunlight or food supply.

It is clear from the above two processes in the plants and animals that energy needed for metabolic reaction can be in the form of electromagnetic wave energy from sunlight or in a bio-chemical energy derived from food intake. This is consistent with the Gibbs Free Energy concept for all chemicals reactions. The Gibbs Free Energy for a chemical reaction includes both the thermal energy (Enthalpy) as well as from Entropy. Therefore for a chemical reaction to proceed, energy may come from thermal energy input, or entropy energy input such as electromagnetic wave excitation energy.

The above is the basic concept of how different living forms obtains their energy for sustaining their live and it is all about how energy can be transferred into the organism from natural source.

For animals, food intake is to provide energy, the basic simple bio-molecules for re-assembling into new cells also energy storage in forms such as fats in the body. This is desirable in nature for animals but it may not be desirable for human being in modern living styles with much lesser opportunity to burn off the storage energy, fats. For human beings modern living lifestyle tend to have excessive food intake and these excessive food energy when stored as excessive fats in our body is causing disorder or sickness. One idea way to solve such a problem is to obtain the energy yet without the worry of excessive storage of energy in the form fats in the body.

The object of the invention is to provide the energy needed for various physiological processes in a mammalian body yet there is no risk of storage of energy in the form of fats in the body.

In order to provide such energy needed by the body it is necessary to have a media which is able to act as an energy carrier yet such energy must be able to dissipate inside the body in a well distributed manner. This is due to the fact that metabolic reactions taking place in various parts of our body instead of just at one localized location. More importantly, after the energy is dispensed in the body, such media should not leave behind the bio-molecules in the body for re-assembling into fats.

One of the idea media is water. Water has the ability to store energy through electromagnetic wave excitation and it is able to transfer its excited energy to a lower energy level forms such as human body. It is also known that human body has more than 60% of water and it is well distributed at different part of the body. By consuming the electromagnetic wave treated water, the excitation energy of the water molecules will be able to dissipate to the various parts of human body for metabolic process. In this process, only net energy is received by the body without bio-molecules and there is no risk for fat build up in the body in this process.

### Summary of the Invention

The present invention has been developed to attain the objects noted above and therefore has a principle object of the provision of a system and method for imparting electromagnetic energy into water using electromagnetic wave having varying frequency, which alters molecular internal vibrational energy of the water to promote the physiological processes including a metabolic process.

Another object of the invention is to provide a system and method for imparting electromagnetic energy into water using electromagnetic wave having varying frequency, which drives the physiological processes without increasing food intake, and which aims to improve metabolism, and hence wellness, by treating drinking water and allowing the body to benefit from the vibrational energy.

A further object of the invention is to provide a system and method for imparting electromagnetic energy into water using electromagnetic wave having varying frequency, which allows dissipation of the energy into all parts of the body that the water is circulated to when the water is consumed.

These and other objects and advantages of the invention are satisfied by providing a system for imparting electromagnetic energy into drinking water or aqueous potables and foods, comprising:
a generator unit for generating a driving signal, the generator unit configured to vary a frequency of the driving signal, wherein the frequency is selected so as to maintain a nature or an activity of microorganisms in living forms;
a power supply unit coupled to the generator unit for providing a voltage current to the generator unit to drive the generation of the driving signal having a varying frequency;
one or more inductor coils coupled to the generator unit to receive the driving signal, such that an electromagnetic field operating with the varying frequency is generated; and
an electromagnetic wave-permeable container for containing the drinking water or the aqueous potables and foods and placed in an area where the electromagnetic wave-permeable container filled with the drinking water or the aqueous potables and foods is subject to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water.

In one embodiment of the invention, the system comprises a plurality of inductor coils in the array form. The inductor coils may be coiled in a flat coil manner or in cylindrical, square, or many other shapes. Preferably, a ferrite core is inserted into each of the inductor coils.

According to the invention, the frequency of the electromagnetic field ranges from about 200 Hz to about 10,000 Hz, preferably from about 200 Hz to about 5,000 Hz. The system electromagnetic field may have a sweeping frequency of 0.1 to 100 Hz, which means one cycle takes 10 second to 0.01 second.

In another preferred embodiment of the invention, the electromagnetic field has a pattern which may be varied in a regular fashion or which may be time-varied. For example the pattern comprises wave form of the electromagnetic field, time interval elapsed between two frequencies to appear, frequency density,

The power supply unit may be a direct current (DC) power source selected from the group consisting of solar panel, battery, DC power supply rectified from alternate current (AC) power source.

The electromagnetic wave-permeable container may be made of a material selected from the group consisting of ceramic, wood, glass, plastics having electromagnetic wave-permeability, and any combination thereof.

The drinking water or the aqueous potables and food receive the effect of the electromagnetic treatment for a few minutes to a few hours, preferably 1 minute to 1 hour, and more preferably 3 minutes to 30 minutes, and most preferably 3 minutes to 10 minutes.

A second aspect of the invention is to provide a method for imparting electromagnetic energy into drinking water or aqueous potables and foods, comprising the steps of:
generating a driving signal to drive generation of an electromagnetic field operating with a varying frequency, wherein the frequency is selected so as to maintain a nature or an activity of microorganisms in living forms;
providing the drinking water or the aqueous potables and foods in an electromagnetic wave-permeable container; and
subjecting the electromagnetic wave-permeable container filled with the drinking water or the aqueous potables and foods to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water.

In some cases, the method of the invention further comprises the step of varying a pattern of the electromagnetic field in a regular fashion or as time increases, the pattern comprising wave form of the electromagnetic field, time interval elapsed between two frequencies to appear, frequency density, sweeping frequency, or any combination thereof.

A third aspect of the invention is to provide a method for providing energy needed for a physiological process in mammalian body, comprising:
generating a driving signal to drive generation of an electromagnetic field operating with a varying frequency, wherein the frequency is selected so as to maintain a nature or an activity of microorganisms in living forms;
providing the drinking water or the aqueous potables and foods in an electromagnetic wave-permeable container;
subjecting the electromagnetic wave-permeable container filled with the drinking water or the aqueous potables and foods to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water; and
consuming the treated drinking water or the treated aqueous potables and foods to dispense the electromagnetic energy into a body fluid.

The physiological process includes metabolic process, regenerative and repairing processes of cells, and fermentation process.

A further aspect of the invention relates to a method for providing bath tub water for body spa, comprising the step of treating the bath tub water with the system of the invention. Advantageously, the system of the invention is configured to be waterproof, and the system is placed inside the bath tub water to energize the water.

The unique feature of the invention is the use of water as a carrier of the electromagnetic wave energy. When the water is energized by the electromagnetic field to acquire the electromagnetic energy, which results in the water molecules altered into a state with higher vibrational energy. Then the water molecules stores vibrational energy which is dissipated into all parts of the mammalian body when the water is consumed. Hence, the energy is transferred to the whole body instead of only a specific area in the body.

To have a better understanding of the invention reference is made to the following detailed description of the invention and embodiments thereof in conjunction with the accompanying drawings.

### Brief Description of the Drawings

Figure 1 is a schematic view of an exemplary system for imparting electromagnetic energy into drinking water or aqueous potables and foods constructed according to the present invention.
Figure 2 is a graph showing surface tension of molecules of control water against time.
Figure 3 is a graph showing surface tension of molecules of water receiving 30 minutes treatment applied by the system of the invention for 30 minutes against time.
Figure 4 is a graph showing surface tension of molecules of water receiving one hour treatment applied by the system of the invention for 1 hour against time.

### Detailed Description of the Preferred Embodiments

While this invention is illustrated and described in preferred embodiments, the system for imparting electromagnetic energy into drinking water or aqueous potables and foods using electromagnetic wave having varying frequency may be produced in many different configurations, sizes, forms and materials.

The time varying pulsating electromagnetic wave has been known to be used extensively in a wide variety of industries. It is the inventor's finding that if the water is indirectly subjected to the time varying pulsating electromagnetic wave, the water would be energized to become a relatively unstable state with higher vibrational energy. Once the energized water is consumed, the energy stored in the water can readily dissipate into a body fluid into which the water flows. The invention is based on this finding.

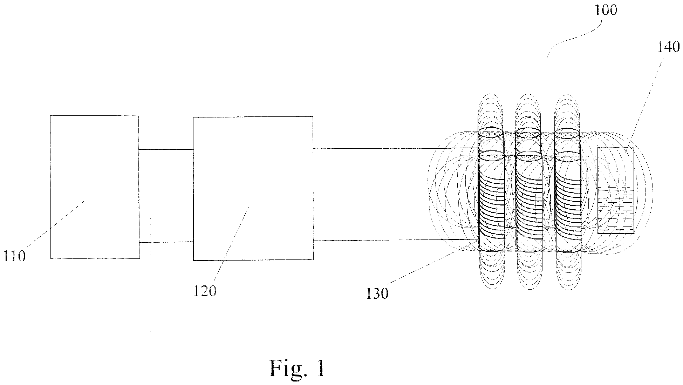

Referring now to the drawings, Fig. 1 provides an exemplary system 100 constructed consistent with the present invention. In this embodiment, the system 100 comprises a power supply unit 110; a generator 120 coupled to the power supply unit 100 for generating a driving signal; a plurality of inductor coils 130 which is coupled to the generator 120; a container 140 for containing water or aqueous potables or aqueous foods and placed in the area affected by the generated electromagnetic field.

The power supply unit 110 is electrically coupled to the generator 120. A direct current (DC) power supply is used in the power supply unit 110. The power supply unit 110 supplies a desired DC driving voltage to the generator 120 enabling the generation of the driving signal. The power supply unit 110 preferably supplies to the generator 120 a driving voltage from a few volts to few hundreds volts, preferably 5V to 50V depending on the actual applications. The power supply unit 110 may be selected from the group consisting of solar panel, battery, DC power supply rectified from alternate current (AC) power source.

The generator 120 can be of any type of means known in the art that is able to generate a driving signal. For example, the generator may be a circuit board or a microcontroller Unit (MCU) programmed to generate the driving signal. The generator 120 is configured to vary a frequency of the driving signal so as to deliver the driving signal having the varying frequency to the inductor coil array. The frequency of the generated driving signal should maintain a nature or an activity of microorganisms in living forms, which will be discussed herein below.

The plurality of inductor coils 130 in this embodiment form a rectangular array. The inductor coil array receives the frequency varying driving signal such that an electromagnetic field having a varying frequency is generated within and around the array. The inductor coil 130 may be coiled in a flat coil manner or in cylindrical, square, or many other shapes. To improve the magnetic flux of the time varying electromagnetic field, a ferrite core may be inserted in the center of each of the inductor coils 130 and the entire coils can be reduced to a very small size. Essentially wires may be used to coil directly on the ferrite core and the coil can be in single layer, Bifilar, multilayers, or ring manners.

The container 140 used in the invention for containing the water or the aqueous potables or foods is electromagnetic wave-permeable to allow the electromagnetic wave to penetrate the container to reach the water or the water present in the aqueous potables and foods. The material of the container 140 is generally non-ferrous or non-metallic, and for example may be selected from the group consisting of ceramic, wood, glass or plastics having electromagnetic wave-permeability. The container 140 with the water is placed in the area that it is subjected to the effects of the electromagnetic field generated by the inductor coil array, thereby the electromagnetic wave is able to penetrate the wall of the container 140 to impart the electromagnetic energy to the water.

Different frequencies of the electromagnetic field are known to influence the nature of microorganism such as bacteria. In general, the frequency greater than about 10,000 Hz would demote or kill the bacteria, the frequency smaller than about 200 Hz would promote the bacteria growth, and the frequency in the range between about 200 Hz and about 10,000 Hz would not promote or demote the bacteria growth.

One of the features of the invention is to subject the water to the electromagnetic field effect so that the water acquires the electromagnetic energy. The desirable frequency of the generated electromagnetic field is preferably between 200 Hz and 10,000 Hz, and more preferably between 200 and 5,000 Hz. The time-varying frequency sweeping time varies from 0.01 seconds to 10 seconds. The electromagnetic field having this frequency range and this sweeping time does not promote or demote the bacteria growth while maintaining the nature and the activity of beneficial bacteria.

The pattern of the electromagnetic field according to the invention may be varied in a regular fashion or randomly. It is preferable to randomly vary the pattern of the electromagnetic field, which has been found to more easily acquire the higher electromagnetic energy. The term "pattern" used herein refers to, for example, the wave form of the electromagnetic field, the time interval elapsed between the appearance of the two frequencies, and the frequency density. Examples of the waveforms are sine wave, square wave, rectangular wave. The elapsed time interval and the frequency density are dependent on the actual requirements, for instance the time interval may be in the range of about 10 to about 100 seconds, and the frequency density may be in the range of about 600 to 5000 each second.

By "randomly" used herein, it is meant that the pattern of the electromagnetic is time-varied and would not be repeated for an extended period of time. The time varying frequency electromagnetic wave is preferable for the invention. The driving signal having a time varying frequency generated by the generator 120 provides that the magnetic and electrical fields are pulsed at a time-varying frequency.

For the production of the desired treatment effect, the operating wave frequency is preferably in the range of 200 Hz to 5,000 Hz, having a sweeping time in the range of 0.1 second to 10 second. The wave form of the time varying frequency electromagnetic wave can be square, triangular, or sinusoidal.

In the plant metabolism, the UV light resonates the excitation of the chlorophylls hence it is able to trigger the process of converting water into H+ proton for ADP → ATP production and leaving oxygen O₂ as waste. Now according to the method of the invention, the water contained in the container 140 is first subject to the electromagnetic field having a time varying frequency in the range from about 200Hz to about 10,000 Hz with a sweeping time varying from 0.01 second to 10 seconds, such that the resonating electromagnetic wave will vibrate the water molecules' electron and change the internal vibration and rotational energy of the water. In this way, the electromagnetic energy is imparted to the water molecules and is reflected in the water internal energy which can be measured by Fourier transform infrared spectroscopy(FTIR), Raman spectroscopy and also the greater fluctuation (larger standard deviation) in water surface tension samples measurement.

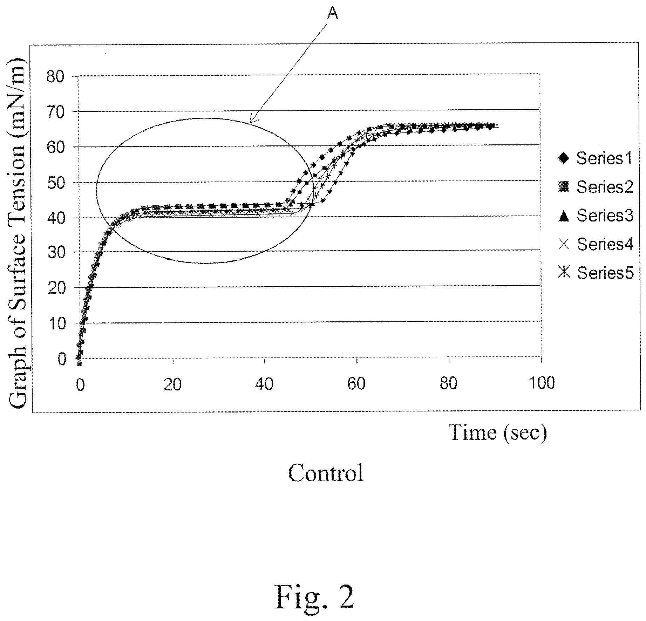

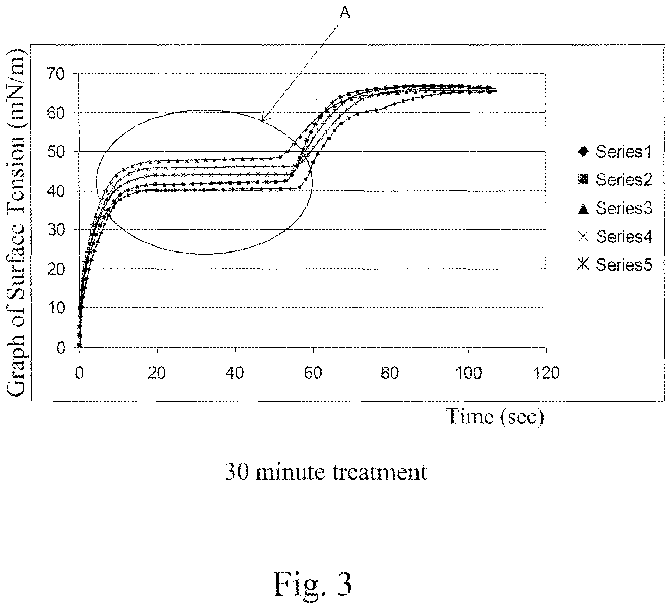

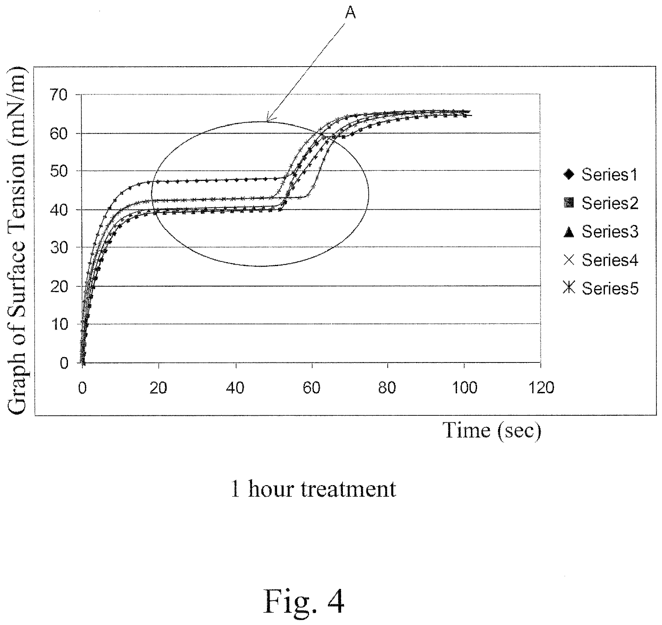

Figs. 2 to 4 show graphs of surface tension (mN/m) against time (second), which were obtained from the tests on the untreated water as a control, the water receiving the electromagnetic wave treatment according to the method of the invention for 30 minutes, and the water receiving the electromagnetic wave treatment according to the method of the invention for 1 hour, wherein Fig. 2 is the result of control water, and Figs. 3 and 4 are results that the water is subjected to the time varying frequency electromagnetic wave treatment for 30 minutes and 1 hour, respectively.

The tests were carried out with the use of a tensionmeter to measure the surface tension of the water according to a method known in the art. Put simply, the needle of the tensionmeter is placed into the water contained in the container at the beginning of the test, and then slowly retracted upwards. The tension exerted on the needle is measured continuously. The plateau of each graph is formed when the needle is just about to separate from the water surface and shows the surface tension of the water. After the needle separates from the water surface and goes out of the water, the tension increases as a result of the force required to move the needle upwards.

A total of five tests were done for each of the control water, the water receiving the 30-minutes treatment and the water receiving the 1-hour treatment to measure the surface tension. Series 1 to 5 in the figures represent the readings of tests 1 to 5 carried out for the same test water at different points of time. The different graphs were prepared on the basis of the obtained readings of the five tests on the same test water.

The readings of the surface tension vary with time. For the control water shown in Fig. 2, there is a small difference in the surface tension results for the readings of the five tests (see the plateaus A indicated by a circle). It is evident that, for the treated water, the plateaus A (indicated by a circle) in Figs. 3 and 4 have a greater deviation in surface tension after the treatment of 30 minutes or 1 hour is received, and the difference in the readings increases with an increase in treatment duration. The treated water by the method of the invention was imparted with the electromagnetic wave energy having time varying frequency, and the energy is transferred to the treated water molecules whose vibrational and dynamic energy increases due to the excitation of the water by the electromagnetic wave having a time-varying frequency. Because the energy of the water molecules is increased dynamically, the surface tension of the water fluctuates relatively widely. These are evidences that the time varying electromagnetic wave energy can excite the water molecule and are imparted to the water. This can be reflected by the test results of relatively big fluctuations of the surface tension of the water receiving the electromagnetic wave treatment.

The water charged with the time varying electromagnetic wave energy is at a higher energy states and is not stable. By the second law of thermodynamics principle, the highly excited water energy will gradually dissipate to the lower energy level surrounding and goes back to its original untreated energy level. This process is a gradual process. As such when the time varying electromagnetic wave treated water is drunk and entered into the human body, the excited time varying wave energy will be able to dissipate to the human body and aid the metabolism in the body as the water is able to migrate to all parts of the body and dissipate the required energy.

In human body anabolic process, energy is drawn from the process of ATP converting to ADP. Now with the time varying electromagnetic wave energy treated water, the required anabolic reaction energy can also be drawn from the time varying electromagnetic wave energy released from the treated water. This energy is in compliance with the exiting anabolic reactions in the body. The same goes with the other physiological activities of human beings which draw the energy from ATP to ADP conversion. All these energy can now be supplemented by the water which received the treatment of the time varying electromagnetic field using the method of the invention. The treated water according to the invention makes it possible that the human body is kept energetic and a better metabolic rate is achieved in the body.

The present invention takes advantage of a post-treatment effect which is to utilize the output energy released from the water receiving the electromagnetic field treatment. When the treated water is consumed, no more energy is charged or enters the water, and there is only energy releasing from the treated water. The post-treatment effect results in elevated level of the vibrational energy of the water molecules' electrons. Such an elevated energy state would tend to release the energy naturally to a lower energy sink, i.e. the body fluid after the treated water is consumed. The water is acting as an energy carrier which dispenses the electromagnetic energy into various parts of the body.

The method and system of the invention can find a wide range of applications, including but not limited to:
- treating drinking water to give it the effect of enhancing metabolism,
- treating water to enhance its cleansing effect,
- treating food and drinks to enhance their taste and remove their astringent taste,
- treating fermented foods to accelerate the fermentation process, and
- treating water used for watering plants to accelerate plant growth.

After the water undergoes the electromagnetic field treatment according to the method of the invention, it becomes disinfected and highly energised water that, when consumed, dissipated energy into the body and improve metabolism.

The intermolecular attractive forces in the treated water undergo dynamic excitation, the water is able to penetrate substances, such as fibers in clothing, thus the cleaning of clothing is greatly improved. At the same time, the stronger intermolecular attractive forces in the water improve its ability to remove lodged foreign substances. These features of the treated water make it excellent for cleaning.

The vibrational energy present in foods increases the rate of metabolic reactions, so the treated aqueous foods are able to react with the enzymes in the saliva more efficiently, allowing the taste buds to pick up stronger taste signals. This means that a smaller amount of sugar is required for the same amount of sweetness to be perceived, helping people, especially diabetics, to reduce their sugar intake for better health. Likewise, less salt is required for the same amount of saltiness to be perceived. With sour foods, the same amount of H+ ions will produce a more stimulating taste. With bitter foods, the taste of bitterness is caused by compounds such as polyphenols and alkaloids and these compounds contain a chemical structure known as a benzene ring which can be broken down by the treated water, causing the bitter taste to be reduced.

The water treated by the method and system of the invention may be utilized as bath tub water for body spa. The energy of the treated water would be transferred into a human body through human skin. The bath tub water is under the electromagnetic wave treatment for a period of time, for example 15 to 30 minutes. Advantageously, the system of the invention is configured to be waterproof, and the system is placed inside the bath tub water to energize the water. The treated bath tub water not only enables oxygenating to the human body to give an invigorating, healthy and more refreshing spa, but also helps to improve metabolism.

### Assays

Assays were carried out for verifying the acquisition of the electromagnetic energy by the water after the electromagnetic field treatment. This can be measured by a charge level in the water.

Specifically, tap water was filled into a spray bottle and subject to the electromagnetic field treatment for about 5 minutes according the method of the invention as described above. As a control, air and untreated water filled into the spray bottle were used in the assays and measured for the charge level.

Then air ion tester was used to measure the charge, and the readings were recorded every 5 seconds. The measurement method is similar to the measure of charge/ions borne in the air using the air ion meter which is well known in the art and therefore will not be described herein.

Below table 1 is the results represented by the unit ions/c.c., showing the number of the charged ions in the test samples.

**Table 1**

| Air | Untreated Water | Treated Water | Treated Water |
|---|---|---|---|
| | | Test 1* | Test 2** |
| 1 x 10⁷ | 4 x 10⁷ | 97 x 10⁷ | 36 x 10⁷ |
| 2 x 10⁷ | 17 x 10⁷ | > 2,000 x 10⁷ | 98 x 10⁷ |
| 0 | 23 x 10⁷ | > 2,000 x 10⁷ | 242 x 10⁷ |
| 2 x 10⁷ | 164 x 10⁷ | > 2,000 x 10⁷ | 301 x 10⁷ |
| 2 x 10⁷ | 203 x 10⁷ | > 2,000 x 10⁷ | 252 x 10⁷ |
| 2 x 10⁷ | 309 x 10⁷ | > 2,000 x 10⁷ | 409 x 10⁷ |
| 2 x 10⁷ | 630 x 10⁷ | 792 x 10⁷*** | 467 x 10⁷ |

| | | | |
|---|---|---|---|
| * The test was done right after the water was treated. ** The test was done about 5 minutes after the 1^{st} test for verification. *** Air ion tester was moved further away from the spray water. | | | |

The data shown in the above table reveals that the energy is significantly increased immediately after the water received the electromagnetic field treatment according to the method of the invention. The charged energy in the water would decrease to a low level in a short time. This suggests that the energy was imparted to the treated water and dispensed quickly to our body after the treated water was consumed. This greatly improves the metabolism process in the human body.

The invention thus provides a system and a method for providing electromagnetic energy into water using the electromagnetic field, which aim to focus on the impartment of electromagnetic energy as molecular (vibrational) internal energy, or entropy, to drive the metabolic process. In this invention, the electromagnetic wave having the desired frequency is used to generate vibrational energy and advance the metabolic process. In addition, the energy impartment into the water is carried indirectly and through the electromagnetic wave-permeable container. These are the most distinguishing and unique features of the invention not known from the prior art technologies.

While the embodiments described herein are intended as exemplary energy imparting system and method using the electromagnetic field, it will be appreciated by those skilled in the art that the present invention is not limited to the embodiments illustrated. Those skilled in the art will envision many other possible variations and modifications by means of the skilled person's common knowledge without departing from the scope of the invention, however, such variations and modifications should fall into the scope of this invention.

## Claims

1. A system for imparting electromagnetic energy into drinking water or aqueous potables and foods, comprising:
a generator unit for generating a driving signal, the generator unit configured to vary a frequency of the driving signal, wherein the frequency is selected so as to maintain a nature or an activity of microorganisms in living forms;
a power supply unit coupled to the generator unit for providing a voltage current to the generator unit to drive the generation of the driving signal;
one or more inductor coils coupled to the generator unit to receive the driving signal, such that an electromagnetic field operating with the varying frequency is generated; and
an electromagnetic wave-permeable container for containing the drinking water or the aqueous potables and foods and placed in an area where the electromagnetic wave-permeable container filled with the drinking water or the aqueous potables and foods is subject to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water.

2. The system as claimed in claim 1, comprising a plurality of inductor coils in the array form.

3. The system as claimed in claim 1 or 2, wherein a ferrite core is inserted into each of the inductor coils.

4. The system as claimed in any one of claims 1 to 3, wherein the frequency of the electromagnetic field ranges from about 200 Hz to about 10,000 Hz, preferably from about 200 Hz to about 5,000 Hz.

5. The system as claimed in any one of claims 1 to 4, wherein the electromagnetic field has a sweeping frequency of 0.1 to 100 Hz.

6. The system as claimed in any one of claims 1 to 5, wherein the electromagnetic field has a pattern which is varied in a regular fashion or which is time-varied, the pattern comprising wave form of the electromagnetic field, time interval elapsed between two frequencies to appear, frequency density, sweeping frequency, or any combination thereof.

7. The system as claimed in any one of claims 1 to 6, wherein the power supply unit is a direct current (DC) power source selected from the group consisting of solar panel, battery, DC power supply rectified from alternate current (AC) power source.

8. A method for imparting electromagnetic energy into drinking water or aqueous potables and foods, comprising the steps of:
generating a driving signal to drive generation of an electromagnetic field operating with a varying frequency, wherein the frequency is selected so as to maintain a nature or an activity of microorganisms in living forms;
providing the drinking water or the aqueous potables and foods in an electromagnetic wave-permeable container; and
subjecting the electromagnetic wave-permeable container filled with the drinking water or the aqueous potables and foods to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water.

9. The method as claimed in claim 8, wherein the frequency of the electromagnetic field ranges from about 200 Hz to about 10,000 Hz, preferably from about 200 Hz to about 5,000 Hz.

10. The method as claimed in claim 8 or 9, wherein the electromagnetic field has a sweeping frequency of 0.1 to 100 Hz.

11. The method as claimed in any one of claims 8 to 10, further comprising the step of varying a pattern of the electromagnetic field in a regular fashion or as time increases, the pattern comprising wave form of the electromagnetic field, time interval elapsed between two frequencies to appear, frequency density, sweeping frequency, or any combination thereof.

12. The method as claimed in any one of claims 8 to 11, wherein the predetermined period of time is 1 minute to 1 hour, and preferably 3 minutes to 15 minutes.

13. A method for providing energy needed for a physiological process in mammalian body, comprising:
generating a driving signal to drive generation of an electromagnetic field operating with a varying frequency, wherein the frequency is selected so as to maintain a nature or an activity of microorganisms in living forms;
providing the drinking water or the aqueous potables and foods in an electromagnetic wave-permeable container;
subjecting the electromagnetic wave-permeable container filled with the drinking water or the aqueous potables and foods to the generated electromagnetic field to allow permeation of the varying frequency electromagnetic wave through the container into the drinking water or the aqueous potables and foods, such that an electromagnetic treatment is induced electro-magnetically in the drinking water or water present in the aqueous potables and food for a predetermined period of time, thereby indirectly imparting electromagnetic energy to the water; and
consuming the treated drinking water or the treated aqueous potables and foods to dispense the electromagnetic energy into a body fluid.

14. The method as claimed in claim 13, wherein the physiological process includes metabolic process, regenerative and repairing processes of cells, and fermentation process.

15. A method for providing bath tub water, comprising the step of treating the bath tub water with the system according to any one of claims 1 to 7 so as to energize the water.
